(19) Europäisches Patentamt
European Patent Office
Office européen des brevets

(11) Veröffentlichungsnummer: **0 048 218**
**A1**

(12) # EUROPÄISCHE PATENTANMELDUNG

(21) Anmeldenummer: **81810369.9**

(22) Anmeldetag: **07.09.81**

(51) Int. Cl.$^3$: **C 07 D 405/14**
C 07 D 411/14, C 07 D 405/06
C 07 D 411/06, A 61 K 31/495
A 61 K 31/445

(30) Priorität: **12.09.80 US 186776**

(43) Veröffentlichungstag der Anmeldung:
**24.03.82 Patentblatt 82/12**

(84) Benannte Vertragsstaaten:
**AT BE CH DE FR IT LI LU NL SE**

(71) Anmelder: **CIBA-GEIGY AG**
**Patentabteilung Postfach**
**CH-4002 Basel(CH)**

(72) Erfinder: **Huebner, Charles Ferdinand, Dr.**
**40 Edgewood Road**
**Chatham New Jersey(US)**

(54) **N-Oxide von N-Oxacyclyl-alkyl-piperidinen, Verfahren zu ihrer Herstellung, pharmazeutische Präparate enthaltend diese Verbindungen, sowie ihre therapeutische Verwendung.**

(57) Die Erfindung betrifft Verbindungen vom Typus der Formel I

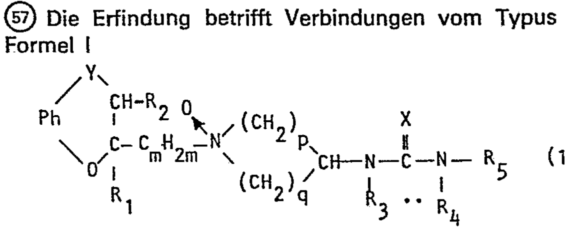

(1) ,

worin Ph unsubstituiertes 1,2-Phenylen oder 1,2-Phenylen, welches durch einen bis drei gleiche oder verschiedene Substituenten der Gruppe bestehend aus Niederalkyl, Niederalkoxy, Niederalkylendioxy, Halogen oder Trifluormethyl substituiert ist, bedeutet, jedes der Symbole $R_1$ und $R_2$ für Wasserstoff oder Niederalkyl steht, jedes der Symbole $R_3$ und $R_4$ Wasserstoff oder Niederalkyl bedeutet oder ($R_3$ + $R_4$) für Ph oder Niederalkylen steht, welches die beiden Stickstoffatome durch 2 bis 4 Kohlenstoffatome trennt, $R_5$ Wasserstoff, Niederalkyl oder HPh bedeutet, X für Oxo steht, Y Epoxy, Epithio oder Sulfinyl steht, m eine ganze Zahl von 1 bis 7 und p und q jeweils 1 bis 3 bedeutet, wobei (p+q) = 4, und Säureadditionssalze dieser Verbindungen. Die Produkte zeigen antidepressive Wirkungen. Sie können durch N-Oxidation der Formel I entsprechenden Ausgangsstoffe hergestellt werden.

EP 0 048 218 A1

CIBA-GEIGY AG
Basel (Schweiz)

4-13328/+

N-Oxide von N-Oxacyclyl-alkyl-piperidinen, Verfahren zu ihrer Herstellung, pharmazeutische Präparate enthaltend diese Verbindungen, sowie ihre therapeutische Verwendung.

In unserem südafrikanischen Patent 79/1276 oder in der äquivalenten, publizierten europäischen Patentanmeldung 4358 sind antidepressive N-Oxacyclyl-alkyl-piperidyl-diazaverbindungen und ihre Säureadditionssalze beschrieben. Es wurde nun gefunden, dass die N-Oxide der obengenannten Verbindungen besonders gute pharmakologische Eigenschaften besitzen.

Die Erfindung betrifft daher diese neuen N-Oxide der N-Oxacyclyl-alkyl-piperidyl-diazaverbindungen der allgemeinen Formel I

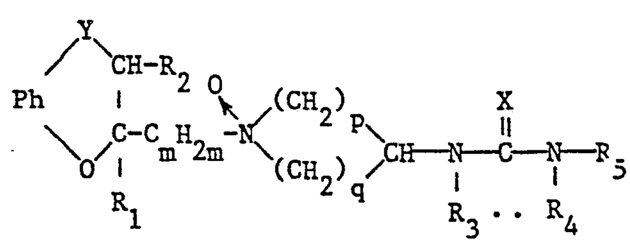

(I) ,

worin Ph unsubstituiertes 1,2-Phenylen oder 1,2-Phenylen, welches durch einen bis drei gleiche oder verschiedene Substituenten der Gruppe bestehend aus Niederalkyl, Niederalkoxy, Niederalkylendioxy, Halogen oder Trifluormethyl substituiert ist, bedeutet, jedes der Symbole $R_1$ und $R_2$ für Wasserstoff oder Niederalkyl steht, jedes der Symbole $R_3$ und $R_4$ Wasserstoff oder Niederalkyl bedeutet oder $(R_3 + R_4)$ für Ph oder Niederalkylen steht, welches die beiden Stickstoffatome durch 2 bis 4 Kohlenstoffatome trennt, $R_5$ Wasserstoff, Niederalkyl oder HPh bedeutet, X für Oxo steht, Y Epoxy, Epithio oder Sulfinyl

bedeutet, m für eine ganze Zahl von 1 bis 7 steht, jedes der Symbole p und q eine ganze Zahl von 1 bis 3 bedeutet, wobei (p + q) die Zahl 4 ist, sowie Säureadditionssalze, insbesondere therapeutisch verwendbare Säureadditionssalze dieser Verbindungen.

Ein 1,2-Phenylenrest Ph ist vorzugsweise unsubstituiert oder monosubstituiert, und seine höchstens drei Substituenten werden durch die folgenden Gruppen illustriert: Niederalkyl, z.B. Methyl, Aethyl, n- oder i-Propyl oder -Butyl; Niederalkoxy, z.B. Methoxy, Aethoxy, n- oder i-Propoxy oder -Butoxy, Niederalkylendioxy, z.B. Methylendioxy, 1,1- oder 1,2-Aethylendioxy, Halogen, z.B. Fluor, Chlor oder Brom oder Trifluormethyl.

Jedes der Symbole $R_1$ bis $R_5$ bedeutet vorzugsweise Wasserstoff, aber auch Niederalkyl, insbesondere Methyl, oder einen anderen oben genannten Niederalkylrest. Das Symbol $R_5$ kann auch für Phenyl stehen, welches unsubstituiert oder substituiert sein kann, wobei die Substituenten diejenigen eines Ph-Restes sind. Die Symbole $R_3$ und $R_4$ können gemeinsam auch den 1,2-Phenylenrest Ph darstellen oder für Niederalkylen stehen, welches die zwei Stickstoffatome insbesondere durch 2 oder 3 Kohlenstoffatome trennt, z.B. für 1,2-Aethylen, 1,2- oder 1,3-Propylen, 1,2-, 1,3- oder 2,3-Butylen. Das Symbol Y bedeutet vorzugsweise Epoxy, aber auch Epithio oder Sulfinyl. Von den genannten ganzen Zahlen ist m vorzugsweise 1 bis 4 und $C_mH_{2m}$ bedeutet vorzugsweise Methylen, 1,1- oder 1,2-Aethylen, 1,2- oder 1,3-Propylen, 1,2- , 1,3- oder 1,4-Butylen, und jedes der Symbole p und q steht vorzugsweise für 2.

Die N-Oxide der Formel I enthalten kein Wasserstoffatom an einem basischen Stickstoffatom und sie werden von der Piperidingruppe, d.h. von ihrem Stickstoffatom in Stellung 1 abgeleitet. Die Verbindungen der allgemeinen Formel I können in Form ihrer Säureadditionssalze, insbesondere therapeutisch verwendbaren Säureadditionssalze, welche z.B. von unten genannten Säuren abgeleitet sind, vorliegen.

Der Ausdruck "nieder" definiert in den oben oder nachfolgend genannten organischen Resten oder Verbindungen solche mit höchstens 7, vorzugsweise 4, insbesondere 1 oder 2 Kohlenstoffatomen.

Die Verbindungen der Erfindung zeigen wertvolle pharmakologische, z.B. antidepressive Wirkungen. Diese können in Tierversuchen, vorzugsweise an Säugetieren, wie Mäusen, Ratten oder Affen, als Testobjekte nachgewiesen werden. Die Verbindungen der Erfindung können ihnen enteral, z.B. oral, oder parenteral, z.B. subkutan, intraperitoneal oder intravenös, z.B. in Form von wässerigen Lösungen oder Stärke enthaltenden Suspensionen, verabreicht werden. Die verwendete Dosis kann in einem Bereich von ungefähr zwischen 0,1 und 100 mg/kg/Tag, vorzugsweise ungefähr 1 und 50 mg/kg/Tag, insbesondere ungefähr 5 und 25 mg/kg/Tag liegen.

Die Verbindungen der Erfindung sind dementsprechend wertvolle Psychostimulantia, z.B. in der Behandlung oder Handhabung von Depressionen oder geringen Gehirnfunktionsstörungen. Die neuen Verbindungen können überdies als Zwischenprodukte zur Herstellung von anderen wertvollen, insbesondere von pharmakologisch wirksamen Verbindungen, eingesetzt werden.

Bevorzugte Verbindungen sind diejenigen der allgemeinen Formel I, worin Ph unsubstituiertes oder durch Niederalkyl, Niederalkoxy, Niederalkylendioxy, Halogen oder Trifluormethyl monosubstituiertes 1,2-Phenylen bedeutet, jedes der Symbole $R_1$ und $R_2$ für Wasserstoff oder Niederalkyl steht, jedes der Symbole $R_3$ und $R_4$ Wasserstoff oder Niederalkyl ist oder $(R_3 + R_4)$ den Rest Ph oder Niederalkylen bedeutet, welches die beiden Stickstoffatome durch 2 oder 3 Kohlenstoffatome trennt, das Symbol $R_5$ für Wasserstoff, Niederalkyl oder HPh steht, X Oxo bedeutet, Y für Epoxy, Epithio oder Sulfinyl steht, m für eine ganze Zahl von 1 bis 4 steht, jedes der Symbole p und q eine ganze Zahl von 1 bis 3 bedeutet, wobei (p + q) die Zahl 4 ist, und Säureadditionssalze, insbesondere therapeutisch verwendbare Säureadditionssalze dieser Verbindungen.

Bevorzugt sind weiter Verbindungen der allgemeinen Formel I, worin Ph unsubstituiertes oder durch Alkyl oder Alkoxy, jeweils mit höchstens 4 Kohlenstoffatomen, Halogen oder Trifluormethyl monosubstituiertes 1,2-Phenylen bedeutet, jedes der Symbole $R_1$, $R_2$ und $R_5$ für Wasserstoff oder Alkyl mit höchstens 4 Kohlenstoffatomen steht, jedes der Symbole $R_3$ und $R_4$ Wasserstoff bedeutet oder $(R_3 + R_4)$ für Alkylen mit 2 bis 4 Kohlenstoffatomen steht, welches die beiden Stickstoffatome durch 2 oder 3 Kohlenstoffatome trennt, X Oxo bedeutet, Y für Epoxy oder Epithio steht, m für eine ganze Zahl von 1 bis 4 steht, jedes der Symbole p und q 2 bedeutet, und Säureadditionssalze,insbesondere therapeutisch verwendbare Säureadditionssalze dieser Verbindungen.

Besonders hervorzuheben sind Verbindungen der allgemeinen Formel II

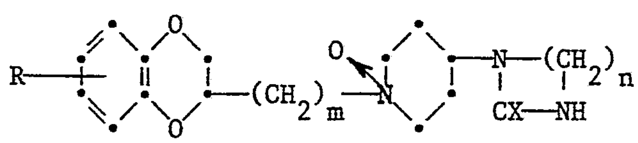

(II) ,

worin R Wasserstoff, Alkyl oder Alkoxy, jeweils mit höchstens 4 Kohlenstoffatomen, Halogen oder Trifluormethyl bedeutet, m für eine ganze Zahl von 1 bis 4 steht, n die ganze Zahl 2 oder 3 bedeutet, und X ist Oxo, und Säureadditionssalze, insbesondere therapeutisch verwendbare Säureadditionssalze dieser Verbindungen.

Die Verbindungen der vorliegenden Erfindung werden nach an sich bekannter Methode dadurch hergestellt, dass man eine Verbindung der allgemeinen Formel III

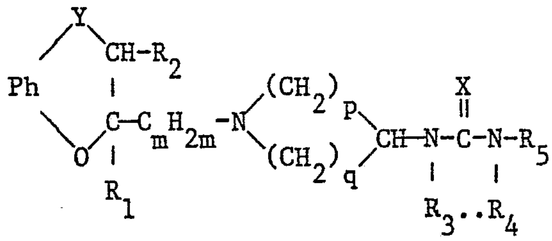

(III) ,

worin alle Symbole die oben angegebenen Bedeutungen haben, zu ihrem N-Oxid der allgemeinen Formel I, oxidiert.

Die Oxidation der Ausgangsstoffe der allgemeinen Formel III wird mit organischen Persäuren, wie niederen Peralkansäuren oder Perbenzoesäuren, z.B. mit Peressig- oder m-Chlorperbenzoesäure, vorzugsweise bei oder über Zimmertemperatur, oder bis 100° mit verdünntem Wasserstoffsuperoxid, in Gegenwart oder Abwesenheit von Niederalkansäuren, z.B. Essigsäure durchgeführt. Man muss, insbesondere mit den genannten Persäuren, vorsichtig arbeiten, um einer Ueberoxidation aufgrund zu langer Reaktionsdauer vorzubeugen.

Die erfindungsgemäss erhaltenen Verbindungen können in an sich bekannter Weise ineinander übergeführt werden. So können z.B. erhaltene Verbindungen, in welchen $R_3$ bis $R_5$ Wasserstoff bedeutet, mit reaktionsfähigen Estern von Niederalkanolen umgesetzt werden. Diese reaktionsfähigen Ester werden von starken anorganischen oder organischen Säuren, in erster Linie von einer Halogenwasserstoffsäure, z.B. Chlorwasserstoff-, Bromwasserstoff- oder Jodwasserstoffsäure, Schwefelsäure oder einer aromatischen Sulfonsäure, z.B. p-Toluolsulfonsäure oder m-Brombenzolsulfonsäure, abgeleitet. Man erhält dabei die entsprechenden N-substituierten Verbindungen. Je nach der verwendeten Mol-Menge des Alkylierungsmittels wird die sukzessive Einführung von $R_3$, $R_4$ und $R_5$ durchgeführt. Verbindungen, in welchen Y ein Schwefelatom bedeutet, können mit milden Oxidationsmitteln zu ihren 4-Oxiden oxidiert werden. Man verwendet z.B. Perjodate, wie Natriumperjodat in polaren Lösungsmitteln und arbeitet bei niederigen Temperaturen, z.B. zwischen ungefähr 0° und Zimmertemperatur.

Schliesslich können die Verbindungen der Erfindung in Form von freien Basen oder als Salze erhalten werden. Eine erhaltene freie Base kann in das entsprechende Säureadditionssalz, vorzugsweise mit Säuren, die therapeutisch verwendbare Säureadditionssalze ergeben oder mit Anionenaustauschern, übergeführt werden. Erhaltene Salze können in die entsprechenden freien Basen, z.B. durch Behandlung mit einer stärkeren Base, wie mit einem Metallhydroxyd oder Ammoniumhydroxyd, basischen Salz oder einem Kationenaustauscher, z.B. mit einem Alkalimetallhydroxyd oder -carbonat, umgewandelt werden. Säuren, die therapeutisch verwendbare Säureadditionssalze ergeben, sind z.B. anorganische Säuren, wie Halogenwasserstoffsäure, z.B. Chlorwasserstoff- oder Bromwasserstoffsäure, oder Schwefel-, Phosphor-, Salpeter- oder Perchlorsäure; oder organische Säuren, wie aliphatische oder aromatische Carbon- oder Sulfonsäuren, z.B. Ameisen-, Essig-, Propion-, Bernstein-, Glykol, Milch-, Aepfel-, Wein-, Zitronen-, Malein-, Fumar-, Hydroxymalein-, Brenztrauben-, Phenylessig-, Benzoe-, 4-Aminobenzoe-, Anthranil-, 4-Hydroxybenzoe-, Salicyl-, 4-Aminosalicyl, Pamoe-, Nikotin-, Methansulfon-, Aethansulfon-, Hydroxyäthansulfon-, Aethylensulfon-, Halogenbenzolsulfon-, Toluolsulfon-, Naphthalinsulfon-, Sulfanil- oder Cyclohexylsulfaminsäure; oder die Ascorbinsäure. Diese oder andere Salze, z.B. die Pikrate, können auch in der Reinigung von freien Basen verwendet werden. Die Basen werden in ihre Salze übergeführt, die Salze abgetrennt und die Basen aus den Salzen freigesetzt.

Infolge der engen Beziehungen zwischen den neuen Verbindungen in freier Form und in Form ihrer Salze sind im vorausgegangenen und nachfolgend unter freien Verbindungen und Salzen sinn- und zweckgemäss gegebenenfalls auch die entsprechenden Salze bzw. freien Verbindungen zu verstehen.

Die für die Herstellung der neuen N-Oxide der vorliegenden Erfindung verwendeten Ausgangsstoffe sind bekannt und sie sind als Endprodukte, z.B. im oben genannten südafrikanischen Patent 79/1276 oder in der europäischen Patentanmeldung 4358 beschrieben. Sie können

nach an sich bekannten Methoden, z.B. dadurch hergestellt werden, dass man einen reaktionsfähigen Ester eines oxacyclischen Alkanols der allgemeinen Formel IV mit einer 1-unsubstituierten Piperidyl-diazaverbindung der allgemeinen Formel V kondensiert:

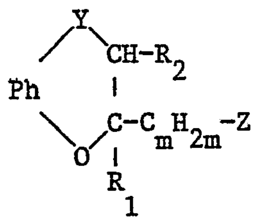

+

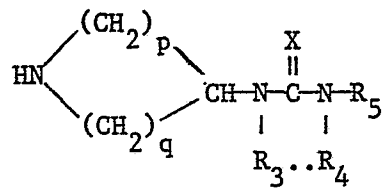

,

(IV) (V)

worin Z eine reaktionsfähige veresterte Hydroxygruppe bedeutet, und die anderen Symbole die oben angegebene Bedeutung haben.

Eine reaktionsfähige veresterte Hydroxygruppe Z ist z.B. eine mit einer starken anorganischen oder organischen Säure, vor allem einer Halogenwasserstoffsäure, z.B. Chlorwasserstoffsäure, Bromwasserstoffsäure oder Jodwasserstoffsäure, Schwefelsäure oder einer aromatischen Sulfonsäure, z.B. p-Toluolsulfonsäure oder m-Brombenzolsulfonsäure veresterte Hydroxygruppe. Die Kondensation wird vorzugsweise in Gegenwart von basischen Kondensationsmitteln, z.B. Alkalimetall- oder Erdalkalimetall-hydroxiden, -carbonaten oder hydrogencarbonaten, wie Natrium, Kalium- oder Calcium-hydroxid oder -carbonat, Alkalimetallhydriden, -niederalkoxiden oder -niederalkanoaten, wie Natriumhydrid, Natriummethylat oder Natriumacetat, oder von organischen tertiären Stickstoffbasen, wie Triniederalkylaminen oder Pyridinen, z.B. Triäthylamin oder Lutidin, durchgeführt.

Ausgangsstoffe und Endprodukte der Formeln I bis V, welche Isomerengemische sind, können nach an sich bekannten Methoden, z.B. durch fraktionierte Destillation, Kristallisation und/oder Chromatographie, in die einzelnen Isomeren getrennt werden. Racemische Produkte können in die optischen Antipoden, z.B. bei Trennung ihrer diastereomeren Salze, z.B. durch fraktionierte Kristallisation der d- oder ℓ-Tartrate, getrennt werden.

Die oben genannten Reaktionen werden nach an sich bekannten Methoden, in Gegenwart oder Abwesenheit von Verdünnungsmitteln, vorzugsweise in solchen, welche gegenüber den Reagenzien inert sind und diese lösen, Katalysatoren, Kondensations- oder Neutralisationsmitteln und/oder in einer inerten Atmosphäre, unter Kühlung, bei Zimmertemperatur oder bei erhöhten Temperaturen, vorzugsweise beim Siedepunkt des verwendeten Lösungsmittels, bei normalem oder erhöhtem Druck durchgeführt.

Die Erfindung betrifft ebenfalls Abänderungen des vorliegenden Verfahrens, wonach ein auf irgendeiner Stufe des Verfahrens erhältliches Zwischenprodukt als Ausgangsmaterial verwendet wird und die verbleibenden Verfahrensschritte durchgeführt werden, oder das Verfahren auf irgendeiner Stufe abgebrochen wird, oder wonach ein Ausgangsmaterial unter den Reaktionsbedingungen gebildet, oder worin ein Ausgangsstoff in Form eines Salzes oder eines optisch reinen Antipoden verwendet wird.

Beim Verfahren der vorliegenden Erfindung werden vorzugsweise solche Ausgangsstoffe verwendet, welche zu den eingangs als besonders wertvoll geschilderten Verbindungen, insbesondere solchen der Formel II führen.

Die pharmakologisch verwendbaren Verbindungen der vorliegenden Erfindung können zur Herstellung von pharmazeutischen Präparaten verwendet werden, welche eine wirksame Menge der Aktivsubstanz zusammen oder im Gemisch mit Trägerstoffen enthalten, die sich zur enteralen oder parenteralen Verabreichung eignen. Vorzugsweise verwendet man Tabletten oder Gelatinekapseln, welche den Wirkstoff zusammen mit Verdünnungsmitteln, z.B. Laktose, Dextrose, Rohrzucker, Mannitol, Sorbitol, Cellulose und/oder Glycin, und Schmiermitteln, z.B. Kieselerde, Talk, Stearinsäure oder Salze davon, wie Magnesium- oder Calciumstearat, und/oder Polyäthylenglykol, aufweisen; Tabletten enthalten ebenfalls Bindemittel, z.B. Magnesiumaluminiumsilikat, Stärke-Paste, Gelatine, Traganth, Methylcellulose, Natriumcarboxymethylcellulose und/oder Polyvinylpyrrolidon, und, wenn erwünscht, Sprengmittel, z.B. Stärken, Agar, Alginsäure oder ein Salz davon, wie Natriumalginat, Enzyme der Bindemittel und/oder Brausemischungen, oder Adsorptionsmittel, Farbstoffe, Geschmackstoffe und Süssmittel. Injizierbare Präparate sind vorzugsweise isotonische wässerige Lösungen oder Suspensionen, und Suppositorien in erster Linie Fettemulsionen oder -suspensionen. Die pharmakologischen Präparate können sterilisiert sein und/oder Hilfsstoffe, z.B. Konservier-, Stabilisier-, Netz- und/oder Emulgiermittel, Löslichkeitsvermittler, Salze zur Regulierung des osmotischen Druckes und/oder Puffer enthalten. Die vorliegenden pharmazeutischen Präparate, die, wenn erwünscht, weitere pharmakologisch wertvolle Stoffe enthalten können, werden in an sich bekannter Weise, z.B. mittels konventioneller Misch-, Granulier- oder Dragierverfahren, hergestellt und enthalten von etwa 0,1 % bis etwa 75 %, insbesondere von etwa 1 % bis etwa 50 % des Aktivstoffes.

Die folgenden Beispiele dienen zur Illustration der Erfindung. Temperaturen werden in Celsiusgraden angegeben, und die Angaben über Teile betreffen Gewichtsteile. Wenn nicht anders definiert, wird das Eindampfen von Lösungsmitteln unter vermindertem Druck, z.B. zwischen ungefähr 15 und 100 mmHg, durchgeführt.

Beispiel 1: Eine Lösung von 7,8 g ℓ-1-[1-[2-(1,4-Benzodioxan-2-yl)-äthyl]-4-piperidyl]-2-imidazolidinon in 100 ml Methylenchlorid wird unter Rühren bei 0° mit einer Lösung von 6,5 g m-Chlorperbenzoesäure in 150 ml Methylenchlorid versetzt. Man lässt das Gemisch über Nacht bei Zimmertemperatur stehen und chromatographiert es auf 200 g basischem Aluminiumoxid. Man eluiert mit Methylenchlorid-Methanol (7:3) und dampft das Eluat ein. Der Rückstand wird in 20 ml Isopropanol aufgenommen, die Lösung mit 2 ml Methansulfonsäure in 5 ml Wasser versetzt und der erhaltene Niederschlag abgetrennt. Man erhält das ℓ-1-[2-(1,4-Benzodioxan-2-yl)-äthyl]-4-(2-oxoimidazolidin-1-yl)-piperidin-1-oxid-methansulfonat-dihydrat, welches unter Dehydratisierung bei 90-94° schmilzt. $[\alpha]_D^{25} = -24,3°$ (c = 2 % in Wasser).

Das Produkt kann man auch durch eine Oxidation mit 30%igem Wasserstoffperoxid in Methanol bei Zimmertemperatur erhalten. Die oben genannte Chromatographie ist hier nicht notwendig.

Man kann 5 g des oben genannten Rückstandes auch in 25 ml Isopropanol aufnehmen und die Lösung mit 2,6 g Cyclohexylsulfaminsäure versetzen. Der Niederschlag wird abgetrennt und aus Wassser umkristallisiert. Man erhält das entsprechende Cyclamat, welches unter Zersetzung bei 180° schmilzt.

Die antidepressive Wirkung des ℓ-1-[2-(1,4-Benzodioxan-2-yl]-äthyl]-4-(2-oxoimidazolidin-1-yl)-piperidin-1-oxid-methansulfonat-dihydrats (=Testsubstanz) kann z.B. durch die noradrenergische Einzeleinheit-Aktivität Testmethode an der Ratte [G. Engberg and T.H. Svensson, Communications in Psychopharmacology, Vol. 4, pp. 23-29 (1980)] wie folgt bestimmt werden:

Männliche CRW Wistar (Charles River) Ratten (280-300 g ) werden mit Chloralhydrat (400 mg/kg i.p.) anästhesiert. Die Tiere werden in ein stereotaxisches Gerät eingespannt, in welchem die extrazellulare

Einheit-Aktivität vom Bereich des locus coeruleus der einen Gehirnseite registriert wird (Glas-Mikroelektrode gefüllt mit 2-molarem Natriumchlorid). Es werden in Intervallen von nacheinanderfolgenden 10 Sekunden extrazellulare Potentiale gezählt und als Histogramm registriert. Die Grundaktivität (baseline firing rate) von jedem untersuchten Neuron wird durch Berechnung der durchschnittlichen Aktivität während 4 Minuten unmittelbar vor der Behandlung mit dem Wirkstoff, ermittelt. Die Wirkung von jeder Dosis der aktiven Substanz wird durch Bestimmung der mittleren Aktivität (firing rate) während einer Minute der maximalen Abweichung vom Kontrollwert, analysiert. In jedem Versuch wird die prozentuale Abweichung vom Kontrollwert bestimmt und ein Mittelwert (± mittlerer Fehler) aus den Gesamtergebnissen bei jeder Dosis berechnet.

Es werden die folgenden Ergebnisse erhalten:

Wirkung der Testsubstanz auf die Aktivität (firing rate) des locus coeruleus Neurons

| | Dosis mg/kg i.v. | Anzahl von getesteten Zellen | Prozentuale Abweichung vom Kontrollwert der Aktivität (firing rate)* |
|---|---|---|---|
| Test-substanz | 10 | 6 | + 76,1 (± 18,1) |
| | 30 | 5 | + 239,4 (± 48,4) |

* Mittelwert ± mittlerer Fehler

Beispiel 2: Die Oxidation von 1-[1-[2-(1,4-Benzodioxan-2-yl)-äthyl]-4-piperidyl]-2-hexahydropyrimidinon, nachfolgende Adsorption der in der Reaktion entstandenen m-Chlorbenzoesäure auf basischem Aluminiumoxid gemäss Beispiel 1 und Eindampfen des Eluats unter vermindertem Druck, ergibt die N-Oxid-Base. Das Hydrochlorid wird durch Hinzufügung von einem Moläquivalent einer 4-normalen äthanolischer Chlorwasserstofflösung zu einer 10%igen Lösung der N-Oxid-Base in Isopropanol hergestellt. Nach vorsichtiger Hinzufügung von Aether bis zur Trübung, erhält man das 1-[2-(1,4-Benzodioxan-2-yl)-äthyl]-4-(2-oxo-hexahydropyrimidin-1-yl)-piperidin-1-oxid-hydrochlorid, welches unter Zersetzung bei 150-155° schmilzt.

Beispiel 3: Die Oxidation von 1-[1-[2-(1,4-Benzoxathian-2-yl)-äthyl]-4-piperidyl]-2-imidazolidinon mit einem Moläquivalent m-Chlorperbenzoesäure und Aufarbeiten des Reaktionsgemisches gemäss Beispiel 1, ergibt nach Hinzufügung von Aether das 1-[2-(1,4-Benzoxathian-2-yl)-äthyl]-4-(2-oxoimidazolidin-1-yl)-piperidin-1-oxid-hydrochlorid als amorphes Material. Nach dem Trocknen im Vakuum erhält man ein Pulver mit unbestimmten Schmelzpunkt. Das Produkt ist einheitlich und ergibt nach Dünnschichtchromatographie auf Silicagel einen $R_f$-Wert von 0,8. Eluierungsmittel: Essigsäureäthylester: Methanol: Ammoniumhydroxid (17:3:3).

Beispiel 4: Eine Lösung von 5,0 g 1-[1-[2-(4-Oxo-1,4-benzoxathian-2-yl)-äthyl]-4-piperidyl]-2-imidazolidinon in 25 ml Methylenchlorid wird bei 5° mit einer Lösung von 4,4 g m-Chlorperbenzoesäure in Methylenchlorid versetzt. Nach 6 Stunden wird die N-Oxid-Base von der m-Chlorbenzoesäure durch Chromatographie auf basischem Aluminiumoxid gemäss Beispiel 1 abgetrennt. Man dampft das Eluat zur Trockene ein, gibt ein Moläquivalent 4-normaler Chlorwasserstoffsäure in Aethanol dazu und dampft wieder zur Trockene ein. Man erhält das gewünschte 1-[2-(4-Oxo-1,4-benzoxathian-2-yl)-äthyl]-4-(2-oxoimidazolidin-1-yl)-piperidin-1-oxid-hydrochlorid als ein

amorphes Pulver. Es wird durch Dünnschichtchromatographie charakterisiert. $R_f$ = 1,5 auf Silicagel. Eluierungsmittel = Essigsäureäthylester: Methanol: Ammoniumhydroxid (17:3:3).

Beispiel 5: Herstellung von 10 000 Tabletten mit einem Gehalt von je 5 mg der aktiven Substanz:

Bestandteile:

| | |
|---|---|
| ℓ-1-[2-(1,4-Benzodioxan-2-yl)-äthyl]-4-(2-oxoimidazolidin-1-yl)-piperidin-1-oxid-methansulfonat-hemihydrat | 50 g |
| Milchzucker | 1157 g |
| Maisstärke | 75 g |
| Polyäthylenglykol 6000 | 75 g |
| Talkpulver | 75 g |
| Magnesiumstearat | 18 g |
| gereinigtes Wasser | q.s |

Verfahren: Sämtliche pulverigen Bestandteile werden mit einem Sieb von 0,6 mm Maschenweite gesiebt. Dann wird der Wirkstoff mit Milchzucker, Talk, Magnesiumstearat und mit der Hälfte der Stärke in einem geeigneten Mischer vermischt. Die andere Hälfte der Stärke wird in 40 ml Wasser suspendiert und die Suspension zur siedenden Lösung von Polyäthylenglykol in 150 ml Wasser gegeben. Die erhaltene Paste wird zu den Pulvern gegeben und gegebenenfalls unter Zugabe einer weiteren Wassermenge granuliert. Das Granulat wird über Nacht bei 35° getrocknet, durch ein Sieb von 1,2 mm Maschenweite getrieben und zu Tabletten von 6,4 mm Durchmesser, welche eine Bruchrille aufweisen, gepresst.

Herstellung von 10 000 Kapseln mit einem Gehalt von je 2,5 mg der aktiven Substanz:

Bestandteile:

| | |
|---|---|
| $\ell$-1-[2-(1,4-Benzodioxan-2-yl)-äthyl]-4-(2-oxoimidazolidin-1-yl)-piperidin-1-oxid-methansulfonat-dihydrat | 25 g |
| Milchzucker | 1875 g |
| Talkpulver | 100 g |

Verfahren: Sämtliche pulverigen Bestandteile werden mit einem Sieb von 0,6 mm Maschenweite gesiebt. Dann wird der Wirkstoff zuerst mit Talk und nacher mit Milchzucker in einem geeigneten Mischer homogenisiert. Gelatine Kapseln Nr. 3 werden mit je 200 mg der erhaltenen Mischung in einer Füllmaschine abgefüllt.

In analoger Weiser werden auch Tabletten und harte Gelatine-Kapseln unter Verwendung von Produkten der anderen Beispiele, hergestellt.

Patentansprüche
(für alle benannten Länder ohne Oesterreich)

1. N-Oxide der N-Oxacyclyl-alkyl-piperidyl-diazaverbindungen der allgemeinen Formel I

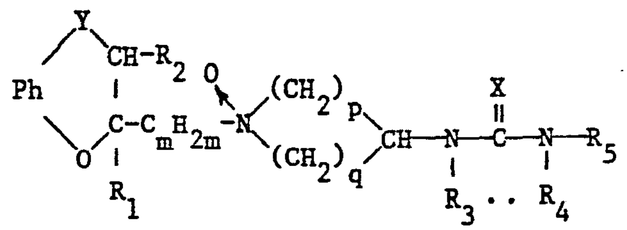

(I) ,

worin Ph unsubstituiertes 1,2-Phenylen oder 1,2-Phenylen, welches durch einen bis drei gleiche oder verschiedene Substituenten der Gruppe bestehend aus Niederalkyl, Niederalkoxy, Niederalkylendioxy, Halogen oder Trifluormethyl substituiert ist, bedeutet, jedes der Symbole $R_1$ und $R_2$ für Wasserstoff oder Niederalkyl steht, jedes der Symbole $R_3$ und $R_4$ Wasserstoff oder Niederalkyl bedeutet oder $(R_3 + R_4)$ für Ph oder Niederalkylen steht, welches die beiden Stickstoffatome durch 2 bis 4 Kohlenstoffatome trennt, $R_5$ Wasserstoff, Niederalkyl oder HPh bedeutet, X für Oxo steht, Y Epoxy, Epithio oder Sulfinyl bedeutet, m für eine ganze Zahl von 1 bis 7 steht, jedes der Symbole p und q eine ganze Zahl von 1 bis 3 bedeutet, wobei (p+q) die Zahl 4 ist, und ihre Säureadditionssalze.

2. Verbindungen nach Anspruch 1, Formel I, worin Ph unsubstituiertes oder durch Niederalkyl, Niederalkoxy, Niederalkylendioxy, Halogen oder Trifluormethyl monosubstituiertes 1,2-Phenylen bedeutet, jedes der Symbole $R_1$ und $R_2$ für Wasserstoff oder Niederalkyl steht, jedes der Symbole $R_3$ und $R_4$ Wasserstoff oder Niederalkyl ist oder $(R_3+R_4)$ den Rest Ph oder Niederalkylen bedeutet, welches die beiden Stickstoffatome durch 2 oder 3 Kohlenstoffatome trennt, das Symbol $R_5$ für Wasserstoff, Niederalkyl oder HPh steht, X Oxo bedeutet, Y für Epoxy, Epithio oder Sulfinyl steht, m für eine ganze Zahl von 1 bis 4 steht, jedes der Symbole p und q eine ganze Zahl von 1 bis 3 bedeutet, wobei (p+q)

die Zahl 4 ist, und ihre Säureadditionssalze.

3. Verbindungen nach Anspruch 1, Formel I, worin Ph unsubstituiertes oder durch Alkyl oder Alkoxy, jeweils mit höchstens 4 Kohlenstoffatomen, Halogen oder Trifluormethyl monosubstituiertes 1,2-Phenylen bedeutet, jedes der Symbole $R_1$, $R_2$ und $R_5$ für Wasserstoff oder Alkyl mit 4 Kohlenstoffatomen steht, jedes der Symbole $R_3$ und $R_4$ Wasserstoff bedeutet oder $(R_3+R_4)$ für Alkylen mit 2 bis 4 Kohlenstoffatomen steht, welches die beiden Stickstoffatome durch 2 oder 3 Kohlenstoffatome trennt, X Oxo bedeutet, Y für Epoxy oder Epithio steht, m für eine ganze Zahl von 1 bis 4 steht, jedes der Symbole p und q 2 bedeutet, und ihre Säureadditionssalze.

4. Verbindungen nach Anspruch 1, welche der allgemeinen Formel II

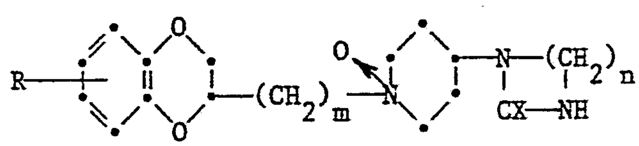

(II)

entsprechen, worin R Wasserstoff, Alkyl oder Alkoxy, jeweils mit höchstens 4 Kohlenstoffatomen, Halogen oder Trifluormethyl bedeutet, m für eine ganze Zahl von 1 bis 4 steht, n die ganze Zahl 2 oder 3 bedeutet, und X ist Oxo, und ihre Säureadditionssalze.

5. Eine Verbindung nach Anspruch 1, welche das 1-[2-(1,2-Benzodioxan-2-yl)-äthyl]-4-(2-oxoimidazolidinon-1-yl)-piperidin-1-oxid ist, seine *ℓ*- und d-Formen und ihre Säureadditionssalze.

6. Pharmazeutische Präparate enthaltend eine Verbindung gemäss einem der Ansprüche 1 bis 5 und ihre Säureadditionssalze, zusammen mit einem pharmazeutischen Trägermaterial.

7. Die im Anspruch 1 genannten Verbindungen zur Anwendung in einem Verfahren zur therapeutischen Behandlung des menschlichen oder tierischen Körpers.

8. Die im Anspruch 1 genannten Verbindungen als antidepressive Mittel.

9. Verwendung der im Anspruch 1 genannten Verbindungen zur Herstellung von pharmazeutischen Präparaten.

10. Verfahren zur Herstellung von den im Anspruch 1 genannten Verbindungen der Formel I und ihren Säureadditionssalzen, dadurch gekennzeichnet, dass man eine Verbindung der allgemeinen Formel III

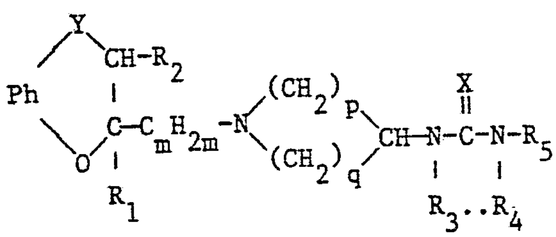

(III) ,

worin alle Symbole die oben angegebenen Bedeutungen haben, zu ihrem N-Oxid der allgemeinen Formel I oxidiert, und, wenn erwünscht, eine erhaltene Verbindung der Formel I in eine andere Verbindung der Erfindung umwandelt, und/oder, wenn erwünscht, eine erhaltene freie Verbindung in ein Säureadditionssalz oder ein Säureadditionssalz in die freie Verbindung oder in ein anderes Säureadditionssalz überführt, und/oder, wenn erwünscht, ein erhaltenes Gemisch von Isomeren oder Razematen in die einzelnen Isomeren oder Razemate auftrennt, und/oder, wenn erwünscht, erhaltene Razemate in die optischen Antipoden aufspaltet.

11. Die nach dem Verfahren des Anspruchs 10 erhältlichen Verbindungen.

Patentansprüche
(für Oesterreich)

1. Verfahren zur Herstellung von neuen N-Oxiden von N-Oxacyclyl-alkyl-piperidyl-diazaverbindungen der allgemeinen Formel I

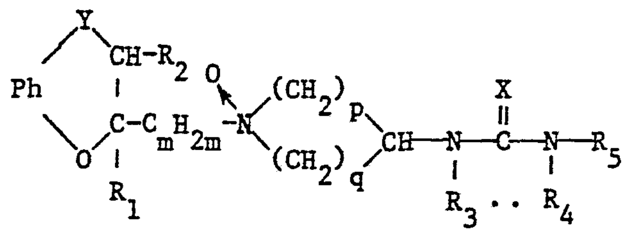

(I) ,

worin Ph unsubstituiertes 1,2-Phenylen oder 1,2-Phenylen, welches durch einen bis drei gleiche oder verschiedene Substituenten der Gruppe bestehend aus Niederalkyl, Niederalkoxy, Niederalkylendioxy, Halogen oder Trifluormethyl substituiert ist, bedeutet, jedes der Symbole $R_1$ und $R_2$ für Wasserstoff oder Niederalkyl steht, jedes der Symbole $R_3$ und $R_4$ Wasserstoff oder Niederalkyl bedeutet oder $(R_3 + R_4)$ für Ph oder Niederalkylen steht, welches die beiden Stickstoffatome durch 2 bis 4 Kohlenstoffatome trennt, $R_5$ Wasserstoff, Niederalkyl oder HPh bedeutet, X für Oxo steht, Y Epoxy, Epithio oder Sulfinyl bedeutet, m für eine ganze Zahl von 1 bis 7 steht, jedes der Symbole p und q eine ganze Zahl von 1 bis 3 bedeutet, wobei (p + q) die Zahl 4 ist, und Säureadditionssalzen dieser Verbindungen, dadurch gekennzeichnet, dass man eine Verbindung der allgemeinen Formel III

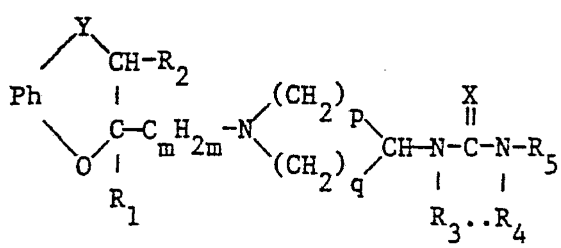

(III) ,

worin alle Symbole die oben angegebenen Bedeutungen haben, zu ihrem N-Oxid der allgemeinen Formel I, oxidiert, und, wenn erwünscht, eine

erhaltene Verbindung der Formel I in eine andere erfindungsgemässe Verbindung umwandelt, und/oder, wenn erwünscht, eine erhaltene freie Verbindung in ein Säureadditionssalz oder ein Säureadditionssalz in die freie Verbindung oder in ein anderes Säureadditionssalz überführt, und/oder, wenn erwünscht, ein erhaltenes Gemisch von Isomeren oder Razematen in die einzelnen Isomeren oder Racemate auftrennt, und/oder, wenn erwünscht, erhaltene Razemate in die optischen Antipoden aufspaltet.

2. Verfahren nach Anspruch 1, dadurch gekennzeichnet, dass man Verbindungen der im Anspruch 1 gezeigten allgemeinen Formel I, worin Ph unsubstituiertes oder durch Niederalkyl, Niederalkoxy, Niederalkylendioxy, Halogen oder Trifluormethyl monosubstituiertes 1,2-Phenylen bedeutet, jedes der Symbole $R_1$ und $R_2$ für Wasserstoff oder Niederalkyl steht, jedes der Symbole $R_3$ und $R_4$ Wasserstoff oder Niederalkyl ist oder $(R_3+R_4)$ den Rest Ph oder Niederalkylen bedeutet, welches die beiden Stickstoffatome durch 2 oder 3 Kohlenstoffatome trennt, das Symbol $R_5$ für Wasserstoff, Niederalkyl oder HPh steht, X Oxo bedeutet, Y für Epoxy, Epithio oder Sulfinyl steht, m für eine ganze Zahl von 1 bis 4 steht, jedes der Symbole p und q eine ganze Zahl von 1 bis 3 bedeutet, wobei (p+q) die Zahl 4 ist, und ihre Säureadditionssalze herstellt.

3. Verfahren nach Anspruch 1, dadurch gekennzeichnet, dass man Verbindungen der im Anspruch 1 angegebenen Formel I, worin Ph unsubstituiertes oder durch Alkyl oder Alkoxy, jeweils mit höchstens 4 Kohlenstoffatomen, Halogen oder Trifluormethyl monosubstituiertes 1,2-Phenylen bedeutet, jedes der Symbole $R_1$, $R_2$ und $R_5$ für Wasserstoff oder Alkyl mit höchstens 4 Kohlenstoffatomen steht, jedes der Symbole $R_3$ und $R_4$ Wasserstoff bedeutet oder $(R_3+R_4)$ für Alkylen mit 2 bis 4 Kohlenstoffatomen steht, welches die beiden Stickstoffatome durch 2 oder 3 Kohlenstoffatome trennt, X Oxo bedeutet, Y für Epoxy oder Epithio steht, m für eine ganze Zahl von 1 bis 4 steht, jedes der Symbole p und q 2 bedeutet, und ihre Säureadditionssalze herstellt.

4. Verfahren nach Anspruch 1, dadurch gekennzeichnet, dass man Verbindungen der allgemeinen Formel II

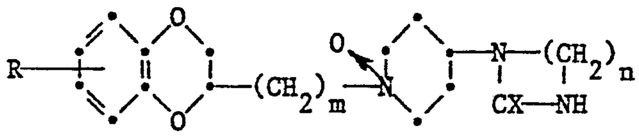

(II) ,

worin R Wasserstoff, Alkyl oder Alkoxy, jeweils mit höchstens 4 Kohlenstoffatomen, Halogen oder Trifluormethyl bedeutet, m für eine ganze Zahl von 1 bis 4 steht, n die ganze Zahl 2 oder 3 bedeutet, und X ist Oxo, und ihre Säureadditionssalze herstellt.

5. Verbindungen nach Anspruch 1, dadurch gekennzeichnet, dass man das 1-[2-(1,4-Benzodioxan-2-yl)-äthyl]-4-(2-oxoimidazolidinon-1-yl]-piperidin-1-oxid, seine ℓ- und d-Formen und ihre Säureadditionssalze herstellt.

6. Verfahren nach Anspruch 1, dadurch gekennzeichnet, dass man das 1-[2-(1,4-Benzodioxan-2-yl)-äthyl]-4-(2-oxo-hexahydropyrimidin-1-yl)-piperidin-1-oxid und seine Säureadditionssalze herstellt.

7. Verfahren zur Herstellung eines pharmazeutischen Präparates, gekennzeichnet durch die Verarbeitung eines erfindungsgemässen Wirkstoffes nach einem der Ansprüche 1 bis 5, mit einem pharmazeutischen Trägermaterial.

8. Verfahren zur Herstellung eines pharmazeutischen Präparates, gekennzeichnet durch die Verarbeitung eines erfindungsgemässen Wirkstoffes nach Anspruch 6, mit einem pharmazeutischen Trägermaterial.

Europäisches Patentamt

# EUROPÄISCHER RECHERCHENBERICHT

Nummer der Anmeldung
EP 81 81 0369

## EINSCHLÄGIGE DOKUMENTE

| Kategorie | Kennzeichnung des Dokuments mit Angabe soweit erforderlich, der maßgeblichen Teile | betrifft Anspruch |
|---|---|---|
| D | EP - A - 0 004 358 (CIBA-GEIGY)<br>* Insgesamt *<br>---- | 1-9, 11 |

**KLASSIFIKATION DER ANMELDUNG (Int Cl ³)**

C 07 D 405/14
411/14
405/06
411/06
A 61 K 31/495
31/445

**RECHERCHIERTE SACHGEBIETE (Int. Cl ³)**

C 07 D 405/14
405/06
411/06
411/14

**KATEGORIE DER GENANNTEN DOKUMENTE**

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| Den Haag | 10-12-1981 | FRANCOIS |